# FASCICULE DE BREVET EUROPEEN

(11) **EP 0 177 393 B1**
(45) Date de publication et mention de la délivrance du brevet: **01.06.1988**
(21) Numéro de dépôt: 85401775.3
(22) Date de dépôt: 13.09.1985
(51) Int. Cl.: C07C 29/136, C07C 31/38, C07C 31/40

(54) **Procédé de préparation de perfluoroalcanols**
Herstellungsverfahren von Perfluoralkanolen
Process for the preparation of perfluoroalkanols

(30) Priorité: 28.09.1984 FR 8414917
(43) Date de publication de la demande: 09.04.1986
(73) Titulaire: RHONE-POULENC CHIMIE, 92408 Courbevoie Cédex (FR)
(72) Inventeur: Cordier, Georges, F-69340 Francheville (FR); Ferlut, Jean-Serge, F-69009 Lyon (FR)
(74) Mandataire: Le Pennec, Magali

(56) Documents cités:
- US-A- 4 232 170
- US-A- 4 273 947

## Description

La présente invention a pour objet un procédé de préparation de perfluoroalcanols; elle concerne plus particulièrement un procédé de préparation de perfluoroalcanols par hydrogénation des esters perfluorés correspondants.

Il est connu selon les brevets US 3 356 747 et US 4 072 726 de préparer des perfluoroalcanols à partir d'esters perfluorés en hydrogénolysant en phase vapeur les esters en présence d'un catalyseur soit à base d'oxyde mixte de chrome et de cuivre, soit à base d'oxyde de Cu seul. Cette hydrogénolyse nécessite un chauffage important des réactifs (200 à 350°C), ce qui entraine des coûts de production trop élevés pour envisager une exploitation industrielle.

Il est aussi connu d'après le brevet français publié sous le n ° 2 060 357 de préparer des perfluoroalcanols par hydrogénolyse d'esters perfluorés dans l'eau en présence d'acide minéral telque lacide phosphorique. L'inconvénient d'utiliser une phase aqueuse est que dans ce cas il se produit une hydrolyse partielle de l'ester avec formation d'acide perfluoré qui dans l'eau est très corrosif per l'appareillage. Ce procédé nest donc pas pour des raisons techniques exploitable industriellement. Il est également connu d'après le brevet européen n ° 36 939 de préparer des perfluoroalcanols par hydrogénolyse d'esters perfluorés correspondants, en présence d'une composition catalytique comprenant un métal du groupe VIII, un métal alcalin et un radical anion choisi parmi les radicaux arène, les alkoxides aliphatiques, à une température d'environ 150°C. La présence d'adjuvants tels que le métal alcalin et le radical anion grèvent le prix de production du trifluoroéthanol.

Il est encore connu d'après le brevet US 4 232 170 un procédé d'hydrogénation catalytique en phase homogène d'esters d'acides carboxyliques à l'aide de catalyseurs qui sont des compositions anioniques d'hydrures de métaux du groupe VIII contenant des ligands organiques à base de phosphore, d'arsenic ou d'antimoine. Ces catalyseurs présentent un caractère de miscibilité avec le milieu réactionnel qui n'apporte aucun avantage industriel mais impose un recyclage indispensable du catalyseur d'un point de vue économique. Ce procédé n'est donc pas pour l'industrie la voie la plus intéressante pour préparer les perfluoroalcanols.

Il est aussi connu d'après le document US 4 273 947 un procédé d'hydrogénation catalytique d'acides carboxyliques en alcools correspondant à l'aide de catalyseurs métalliques choisis parmi le rhodium ou l'irridium, dans un milieu constitué des esters de ces acides. Ce document ne peut donc en aucun cas inciter l'homme de l'art à hydrogéner ces esters qui sont considérés comme inertes par le présent document.

Aucun des procédés préalablement décrit ne permet une exploitation industielle soit parce que la technique est trop onéreuse, soit parce que l'appareillage peut être corrodé.

La présente invention a su vaincre ces problèmes, elle a pour objet un procédé de préparation de perfluoroalcanols en milieu hétérogène caractérisé en ce qu'on met en présence, en phase liquide, un ester d'un acide perfluoré répondant à la formule générale (1): 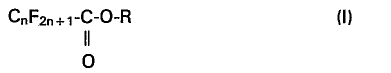 dans laquelle:
n est supérieur ou égal à 1
R est choisi parmi les groupes alkyle, alkylphényle, cycloalkyle, phényle, halogéno alkyle, halogénoal- kylphényle, halogénocycloalkyle, halogénophényle, éventuellement, en mélange avec un ester d'un acide non perfluoré de formule générale (II): 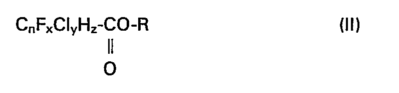 dans laquelle:
   n est un nombre entier supérieur ou égal à 1
   x est un nombre entier compris entre 0 et 2n
   y est un nombre entier compris entre 0 et 2n + 1
   z est un nombre entier compris entre 0 et 2n + 1
   la somme x + y + z est égale à 2n + 1
   R a la même signification que dans la formule (1) avec de l'hydrogène sous une pression totale comprise entre 1 et 300 bars en présence d'un catalyseur solide à base d'au moins un métal choisi parmi le nickel, le cobalt, le cuivre et les métaux de la mine du platine.

Dans la formula (I) et (II) n est de préférence égal ou supérieur à 1 et égal ou inférieur à 12.

Parmi les composés de formule (I), on peut citer: le trifluoroacétate d'éthyle, le trifluoroacétate de phényle, le trifluoroacétate de trifluoroéthyle. Il est préféable d'utiliser les composés de formule (1) dans laquelle R représente un groupe 1,1-dihydroperfluo- roalcane et encore plus préférentiellement un groupe -CH₂-CnF2n+l.

L'ester de l'acide perfluoré préfère selon l'invention est le trifluoroacétate de trifluoroéthyle.

Selon une mise en oeuvre préférée de l'invention, le nickel, le cobalt ou le cuivre sont de type Raney.

Le catalyseur utilisé selon le procédé de l'invention peut être choisi parmi les catalyseurs à base de métaux de la mine du platine. Ces métaux peuvent notamment être choisis parmi le ruthénium, le rhodium, l'irridium, le platine et le palladium.

Ces métaux peuvent être utilisés à l'état métallique, sous forme d'un oxyde, d'un sel tel que par exemple les chromites ou sous forme d'un mélange d'entre eux. Ils peuvent être, de plus, déposés sur support ou pas. On peut utiliser tout support inerte dans les condititons de la réaction comme le charbon, la silice ou l'alumine. On préférera tout particulièrement utiliser le charbon.

On préfère utiliser parmi les catalyseurs, un catalyseur à base de ruthénium ou de rhodium. Ce catalyseur est de préférence déposé sur charbon.

On met, en oeuvre, de préférence, une quantité de métal déposée sursupport, inférieure à 20% en poids et de préférence comprise entre 1 et 10% en poids et encore, plus préférentiellement, d'environ 5% en poids.

La quantité de catalyseur exprimée en poids de métal mise en oeuvre est de préférence inférieure à 10% en poids par rapport à l'ester de l'acide perfluoré. Pour les métaux de la mine du platine, on utilise plus préférentiellement, moins de 2% de métal et encore plus préférentiellement moins de 1 % de métal. Pour le nickel de Raney, on utilise de préférence entre 1 et 5% en poids de métal.

On opère de préférence à une température comprise entre la température ambiante et 300°C et encore plus préférentiellement entre 80 et 150°C.

Une pression totale comprise entre 5 et 150 bar est avantageuse pour la mise en oeuvre de l'invention, une pression comprise entre 15 et 50 bars est préférée.

La réaction peut notamment avoir lieu en présence de solvants inertes à l'hydrogénation tels que les alcanols, les cycloalcanols, les alcanes, les cycloal- canes, les éthers, les esters non fluorés. On peut citer comme exemple de tels solvants: le trifluoroéthanol, le cyclohexanol, le diisopropyléther, les coupes pétrolières correspondant à des alcanes d'environ 6 à 12 atomes de carbone, les esters de l'acide acétique tel que l'acétate de butanol secondaire. Lorsque l'on met en oeuvre la réaction en présence de solvant, on utilise de préférence le trifluoroéthanol, mais dans le cas des esters les plus légers, on opère de préférence sans solvant.

Ce procédé permet en outre, si l'on met en oeuvre un ester d'un acide perfluoré obtenu par exemple par fluoration incomplète de l'acide perchloré correspondant et contenant des produits répondant à la formule générale (II), d'éviter la formation des alcools non perfluorés tel que par exemple le monofluoro- éthanol dont la toxicité même à dose très faible est extrêmement élevée. En effet, lors de l'hydrogénation en présence des catalyseurs selon l'invention, les esters d'acides non perfluorés de formule (II) sont totalement transformés en acide fluorhydrique, acide chlorhydrique et en composés organiques non toxiques.

Les acides formés ont pour effet de ralentir la réaction d'hydrogénation. Aussi, pour éviter cet effet néfaste, on ajoute avantageusement une base. Si la réaction a lieu en l'absence de solvant ou en présence de solvants organiques, on ajoute de préférence une base organique choisie parmi les amines tertiaires, les hydroxydes d'ammonium quaternaires. La réaction peut avoir lieu en présence d'une quantité d'eau, dont le rapport molaire par rapport à l'ester est inférieur à 10% ce qui permet l'ajout d'une base minérale telle que la soude, la potasse, la lithine, les carbonates. On préfère dans ce cas ajouter la soude ou la potasse.

L'invention est particulièrement indiquée pour l'obtention de trifluoroéthanol par hydrogénation du trifluoroacétate de trifluoroéthyle. Le trifluoroéthanol est utilisé comme intermédiaire de synthèse dans l'industrie pharmaceutique ou phytosanitaire.

L'invention va être plus complètement décrite à l'aide des exemples suivants qui ne doivent pas être considérés comme limitatifs de l'invention.

### Exemple 1

Dans un autoclave de 125 cm³ en Hastelloy 8₂ on charge:
- 20 g de trifluoroacétate de trifluoroéthyle
- 0,8 g d'un catalyseur au ruthénium déposé à 5% sur du charbon.

L'autoclave est purgé de l'air qu'il contient par de l'azote puis l'azote est à son tour éliminé par de l'hydrogène. On pressurise l'autoclave par 100 bars d'hydrogène et on chauffe à 100°C. On laisse réagir 3 heures en maintenant cette température et une pression totale de 100 bars. L'analyse de la phase liquide montre que le taux de transformation du trifluoroacétate de trifluoroéthyle est de 98% et le rendement en trifluoroéthanolest de 95%.

### Exemple 2

Cet exemple est réalisé dans le même appareillage et selon le procédé décrit dans l'exemple 1 mais en présence de:
- 18 g de trifluoroacétate de trifluoroéthyle
- 0,3 g d'un catalyseur au rodhium déposé à 5% sur charbon et dans les conditions réactionnelles suivantes:
- pression totale: 19 bars d'hydrogène
- température: 90°C
- durée de la réaction: 6 heures.

On obtient un taux de transformation du trifluoroacétate de trifluoroéthyle de 95% et un rendement en trifluoroéthanol de 96%.

### Exemple 3

Cet exemple est réalisé dans le même appareillage et selon le procédé décrit dans l'exemple 1 mais en présence de:
- 18 g de trifluoroacétate de trifluoroéthyle
- 0,3 g d'un catalyseur au rodhium déposé à 5% sur charbon dans les conditions réactionnelles suivantes:
- pression totale: 50 bars d'hydrogène
- température: 90°C
- durée de la réaction: 4 heures.

On obtient un taux de transformation du trifluoroacétate de trifluoroéthyle de 97% et un rendement en trifluoroéthanol de 96%.

### Exemple 4

Cet exemple est réalisé dans le même appareillage et selon le procédé décrit dans l'exemple 1 mais en présence de:
- 18 g de trifluoroacétate de trifluoroéthyle
- 0,3 g d'un catalyseur au ruthénium déposé à 5% sur charbon dans les conditions réactionnelles suivantes:
- pression totale: 15 bars
- température: 90°C
- durée: 16 H 30.

On obtient un taux de transformation du trifluoroacétate de trifluoroéthyle de 100% et un rendement en trifluoroéthanol de 89%.

### Exemple 5

Cet exemple est réalisé dans le même appareillage et selon le procédé décrit dans l'exemple 1 mais en présence de:
- 12 g de trifluoroacétate de trifluoroéthyle
- 0,5 g d'un catalyseur à l'irridium déposé à 5% sur charbon dans les conditions suivantes:
- pression totale: 100 bars
- température: 100°C
- durée: 6 heures.

On obtient un taux de transformation du trifluoroacétate de trifluoroéthyle de 96% et un rendement en trifluoroéthanol de 90%.

### Exemple 6

Cet exemple est réalisé dans le même appareillage et selon le procédé décrit dans l'exemple 1 mais en présence de:
- 12 g de trifluoroacétate de trifluoroéthyle
- 0,5 g d'un catalyseur à l'irridium déposé à 5% sur charbon dans les conditions suivantes:
- pression totale: 100 bars
- température: 100°C
- durée: 15 heures.

On obtient un taux de transformation du trifluoroacétate de trifluoroéthyle de 90% et un rendement en trifluoroéthanol de 94%.

### Exemple 7

Cet exemple est réalisé dans le même appareillage et selon le procédé décrit dans l'exemple 1 mais en présence de:
- 43 g de trifluoroacétate de trifluoroéthyle
- 1,92 g d'un catalyseur au palladium à 10% sur charbon dans les conditions suivantes:
- pression totale: 100 bars
- température: 150°C
- durée: 21 heures.
On obtient un taux de transformation du trifluoroacétate de trifluoroéthyle de 45% et un rendement en trifluoroéthanol de 92%.

### Exemple 8

Cet exemple est réalisé dans le même appareillage et selon le procédé décrit dans l'exemple 1 mais en présence de:
- 18 g de trifluoroacétate d'éthyle
- 1,6 g d'un catalyseur au ruthénium déposé à 5% sur charbon dans les conditions réactionnelles suivantes:
- pression totale: 100 bars
- température: 150°C
- durée: 5 heures.
On obtient un taux de transformation du trifluoroacétate d'éthyle de 100% et un rendement en trifluoroéthanol de 89%.

### Exemple 9

Cet exemple est réalisé dans le même appareillage et selon le procédé décrit dans l'exemple 1 mais en présence de:
- 18 g de trifluoroacétate d'éthyle
- 1,6 g d'un catalyseur à l'irridium déposé à 5% sur charbon dans les conditions réactionnelles suivantes:
- pression totale: 100 bars
- température: 150°C
- durée: 5 heures.

On obtient un taux de transformation du trifluoroacétate d'éthyle de 30% et un rendement en trifluoroéthanol de 97%.

### Exemple 10

Cet exemple est réalisé dans le même appareillage et selon le procédé décrit dans l'exemple 1 mais en présence de:
- 37 g de trifluoroacétate de phényle
- 1,92 g d'un catalyseur au rhodium déposé à 5% sur charbon dans les conditions réactionnelles suivantes:
- pression totale: 100 bars
- température: 90°C
- durée: 15 H 30.

On obtient un taux de transformation du trifluoroacétate de phényle de 100% et un rendement en trifluoroéthanol de 98%.

### Exemple 11

Cet exemple est réalisé dans le même appareillage et selon le procédé décrit dans l'exemple 1 mais en présence de:
- 33 g de trifluoroacétate de cyclohexyle
- 1,92 g d'un catalyseur au rhodium déposé à
5% sur charbon dans les conditions réactionnelles suivantes:
- pression totale: 100 bars
- température: 100°C
- durée: 22 H 30.
On obtient un taux de transformation du trifluoroacétate de cyclohexyle de 100% et un rendement en trifluoroéthanol de 91 %.

## Revendications

1. Procédé de préparation en milieu hétérogène de perfluoroalcanols caractérisé en ce qu'on met en présence, en phase liquide, un ester d'un acide perfluoré répondant à la formule générale (1): 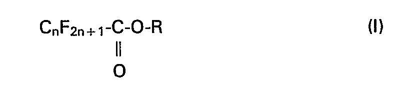 dans laquelle:
n est supérieur ou égal à 1
R est choisi parmi les groupes alkyle, alkylphényle, cycloalkyle, phényle, halogéno alkyle, halogénoal- kylphényle, halogénocycloalkyle, halogénophényle, éventuellement, en mélange avec un ester d'un acide non perfluoré de formule générale (II): 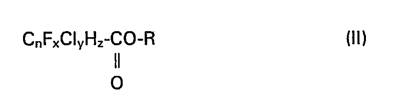 dans laquelle:
n est un nombre entier supérieur ou égal à 1
x est un nombre entier compris entre 0 et 2n
y est un nombre entier compris entre 0 et 2n + 1
z est un nombre entier compris entre 0 et 2n + 1
la somme x + y + z est égale à 2n + 1
R a la même signification que dans la formule (I) avec de l'hydrogène sous une pression totale comprise entre 1 et 300 bars en présence d'un catalyseur solide à base d'au moins un métal choisi parmi le nickel, le cobalt, le cuivre et les métaux de la mine du platine.

2. Procédé selon la revendication 1, caractérisé en ce que, dans la formule (1) et (II), n est égal ou supérieur à 1 et égal ou inférieur à 12.

3. Procédé selon la revendication 1, caractérisé en ce que dans la formule (I) Restun groupe 1,1-dihy- droperfluoroalcane.

4. Procédé selon l'une quelconque des revendications 1 à 3, caractérisé en ce que dans la formule générale (I) R est un radical qui répond à la formule générale -CH2-C"Fzn+

5. Procédé selon la revendication 4, caractérisé en ce que l'ester d'un acide perfluoré est le trifluoroacétate de trifluoroéthyle.

6. Procédé selon la revendication 1, caractérisé en ce que le catalyseur est composé de nickel, de cobalt ou de cuivre du type Raney.

7. Procédé selon la revendication 1, caractérisé en ce que le métal de la mine du platine est choisi parmi le ruthénium, le rhodium, l'irridium, le platine et le palladium.

8. Procédé selon la revendication 1, caractérisé en ce que le métal peut être utilisé sous forme métallique, sous forme d'un oxyde ou d'un sel ou d'un mélange d'entre eux.

9. Procédé selon les revendications 5 ou 6, caractérisé en ce que le métal de la mine du platine est déposé sur un support choisi parmi le charbon, la silice ou l'alumine.

10. Procédé selon les revendications 1 à 5 et 7 à 9, caractérisé en ce que le catalyseur est à base de ruthénium ou de rhodium déposé sur charbon.

11. Procédé selon l'une quelconque des revendications précédentes, caractérisé en ce que l'on met en oeuvre de 1 à 10% en poids de catalyseur exprimé en métal par rapport à l'ester de l'acide perfluoré.

12. Procédé selon l'une quelconque des revendications précédentes, caractérisé en ce que la température de la réaction est comprise entre la température ambiante et 300°C.

13. Procédé selon la revendication 12, caractérisé en ce que la température de la réaction est comprise entre 80 et 150°C.

14. Procédé selon l'une quelconque des revendications précédentes, caractérisé en ce que la pression totale est comprise entre la pression atmosphérique et 250 bars et de préférence entre 5 et 150 bars et encore plus préférentiellement entre 15 et 50 bars.

15. Procédé selon l'une quelconque des revendications précédentes, caractérisé en ce qu'on opère en présence d'un solvant.

16. Procédé selon la revendication 15, caractérisé en ce que le solvant est le trifluoroéthanol.

17. Procédé selon la revendication 1, caractérisé en ce que quand le composé de formule Il est présent, on ajoute une base.

18. Procédé selon la revendication 17, caractérisé en ce que la base est choisie parmi les bases organiques ou minérales.

19. Procédé selon la revendication 18, caractérisé en ce que la base minérale est mise en solution dans une quantité d'eau telle que le rapport molaire eau à l'ester est inférieur à 10%.

## Patentansprüche

1. Verfahren zur Herstellung von Perfluoralkanolen in heterogenem Milieu, dadurch gekennzeichnet, dass man in flüssiger Phase einen Ester einer perfluorierten Säure entsprechend der allgemeinen Formel (I): 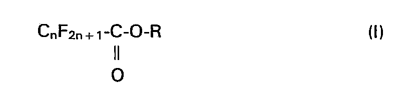 in der
n eine ganze Zall grösser oder gleich 1 ist,
R ausgewählt wird aus den Resten Alkyl, Alkylphenyl, Cycloalkyl, Phenyl, Halogenalkyl, Halogenalkylphenyl, Halogencycloalkyl, Halogenphenyl, gegebenenfalls im Gemisch mit einem Ester einer nicht perfluorierten Säure der allgemeinen Formel (II): 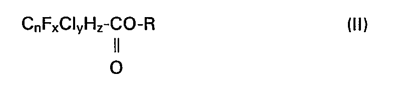 in der
n eine ganze Zahl grösser oder gleich 1 ist,
x eine ganze Zahl zwischen 0 und 2n ist,
y eine ganze Zahl zwischen 0 und 2n + 1 ist,
z eine ganze Zahl zwischen 0 und 2n + 1 ist,
die Summe x + y = z gleich 2n + 1 ist,
R die gleiche Bedeutung hat wie in Formel (I) mit Wasserstoff umsetzt bei einem Gesamtdruck zwischen 1 und 300 bar in Gegenwart eines festen Katalysators auf Basis wenigstens eines Metalls ausgewählt aus Nickel, Kobalt, Kupfer und den Platinmetallen.

2. Verfahren nach Anspruch 1, dadurch gekennzeichnet, dass in der Formel (I) und (11) n grösser oder gleich 1 und kleiner oder gleich 12 ist.

3. Verfahren nach Anspruch 1, dadurch gekennzeichnet, dass in der Formel (I) R ein 1, 1-Dihydroperfluoralkanrest ist.

4. Verfahren nach einem der Ansprüche 1 bis 3, dadurch gekennzeichnet, dass in der allgemeinen Formel (I) R ein Rest ist, der der allgemeinen Formel -CH_{Z}-CₙF₂ₙ+i entspricht.

5. Verfahren nach Anspruch 4, dadurch gekennzeichnet, dass der Ester einer perfluorierten Säure Trifluorethyltrifluoracetat ist.

6. Verfahren nach Anspruch 1, dadurch gekennzeichnet, dass der Katalsator aus Nickel, Kobalt oder Kupfer vom Raney-Typ besteht.

7. Verfahren nach Anspruch 1, dadurch gekennzeichnet, dass das Platinmetall ausgewählt wird aus Ruthenium, Rhodium, Iridium, Platin und Palladium.

8. Verfahren nach Anspruch 1, dadurch gekennzeichnet, dass das Metall in metallaischer Form, in Form eines Oxids oder eines Salzes oder eines Gemisches von diesen verwendet wird.

9. Verfahren nach Anspruch 5 oder 6, dadurch gekennzeichnet, dass das Platinmetall auf einen Träger ausgewählt aus Kohle, Siliciumdioxid oder Aluminiumoxid aufgebracht ist.

10. Verfahren nach den Ansprüchen 1 bis 5 und 7 bis 9, dadurch gekennzeichnet, dass der Katalysator auf Basis von Ruthenium oder Rhodium ist aufgebracht auf Kohle.

11. Verfahren nach einem der vorhergehenden Ansprüche, dadurch gekennzeichnet, dass man 1 bis 10 Gew.-% Katalysator ausgedrückt als Metall bezogen auf den Ester der perfluorierten Säure einsetzt.

12. Verfahren nach einem der vorhergehenden Ansprüche, dadurch gekennzeichnet, dass die Temperatur der Reaktion zwischen der Raumtemperatur und 300°C liegt.

13. Verfahren nach Anspruch 12, dadurch gekennzeichnet, dass die Temperatur der Reaktion zwischen 80 und 150°C liegt.

14. Verfahren nach einem der vorhergehenden Ansprüche, dadurch gekennzeichnet, dass der Gesamtdruck zwischen dem atmosphärischen Druck und 250 bar und vorzugsweise zwischen 5 und 150 bar und weiter vorzugsweise zwischen 15 und 50 bar beträgt.

15. Verfahren nach einem der vorhergehenden Ansprüche, dadurch gekennzeichnet, dass man in Gegenwart eines Solvens arbeitet.

16. Verfahren nach Anspruch 1 5, dadurch gekennzeichnet, dass das Solvens Trifluoroethanol ist.

17. Verfahren nach Anspruch 1, dadurch gekennzeichnet, dass man, wenn die Verbindung der Formel 11 vorliegt, eine Base zugibt.

18. Verfahren nach Anspruch 17, dadurch gekennzeichnet, dass die Base ausgewählt wird aus den organischen oder mineralischen Basen.

19. Verfahren nach Anspruch 18, dadurch gekennzeichnet, dass die Mineralbase in einer solchen Menge Wasser gelöst wird, dass das molare Verhältnis Wasser : Ester unterhalb von 10% liegt.

## Claims

1. Process for the preparation of perfluoroalka- nois in a hererogenous medium, characterized in that an ester of a perfluorinated acid corresponding to the general formula (I): 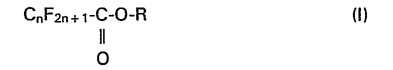 in which
n is an integer equal to or greater than 1 and
R is chosen from among alkyl, alkylphenyl, cycloalkyl, phenyl, halogenoalkyl, halogenoalkylphenyl, halogenocycloalkyl and halogenophenyl groups, optionally mixed with an ester of a non-perfluorinated acid of the general formula (II): 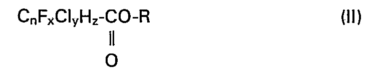 in which
n is an integer greater than or equal to1,
x is an integer between 0 and 2n,
y is an integer between 0 and 2n + 1,
z is an integer between 0 and 2n + 1,
the sum of x + y + z is equal to 2n + 1 and
R has the same meaning as in formula (I) is brought into contact, in a liquid phase, with hydrogen at a total pressure of between 1 and 300 bars in the presence of a solid catalyst based on at least one metal chosen from among, nickel, cobalt, copper and the metals of the platinum group.

2. Process according to claim 1, characterized in that in the formula (I) and (11) n is equal to or greater than 1 and equal to or less than 12.

3. Process according to claim 1, characterized in that in the formula (I) R is a 1,1-dihydroperfiuoroai- kane group.

4. Process according to any one of claims 1 to 3, characterized in that in the general formula (I) R is a radical which corresponds to the general formula -CH₂-CₙF₂ₙ₊1.

## Claims

5. Process according to claim 4, characterized in that the ester of a perfluorinated acid is trifluoroethyl trifluoroacetate.

6. Process according to claim 1, characterized in thatthe catalyst is composed of nickel, cobalt or copper of the Raney type.

7. Process according to claim 1, characterized in that the metal of the platinum group is chosen from among ruthenium, rhodium, iridium, platinum and palladium.

8. Process according to claim 1, characterized in that the metal can be used in the metallic form, in the form of an oxide or a salt, or in the form of a mixture of these.

9. Process according to claims 5 or 6, characterized in that the metal of the platinum group is deposited on a carrier chosen from among charcoal, silica or alumina.

10. Process according to claims 1 to5 and 7 to 9, characterized in that the catalyst is based on ruthenium or rhodium deposited on charcoal.

11. Process according to any one of the preceding claims, characterized in that from 1 to 10% by weight of catalyst, expressed as metal based on the perfluorinated acid ester, is employed.

12. Process according to any one of the preceding claims, characterized in that the reaction temperature is between ambient temperature and 300°C.

13. Process according to claim 12, characterized in that the reaction temperature is between 80 and 150°C.

14. Process according to any one of the preceding claims, characterized in that the total pressure is between atmospheric pressure and 250 bars and preferably between 5 and 150 bars and more preferentially still between 15 and 50 bars.

15. Process according to any one of the preceding claims, characterized in that it is carried out in the presence of a solvent.

16. Process according to claim 15, characterized in that the solvent is trifluoroethanol.

17. Process according to claim 1, characterized in that when the compound of formula II is present, a base is added.

18. Process according to claim 17, characterized in thatthe base is chosen from among organic or inorganic bases.

19. Process according to claim 18, characterized in that the inorganic base is dissolved in an amount of water such that the molar ratio of water to ester is less than 10%.
